(19) Europäisches Patentamt
European Patent Office
Office européen des brevets

(11) Publication number: **0 499 672 A1**

(12) **EUROPEAN PATENT APPLICATION**

(21) Application number: **91102534.4**

(51) Int. Cl.5: **C08L 33/14**, C08F 220/28

(22) Date of filing: **21.02.91**

(43) Date of publication of application: **26.08.92 Bulletin 92/35**

(84) Designated Contracting States: **BE CH DE FR GB IT LI NL**

(71) Applicant: **SANYO CHEMICAL INDUSTRIES, LTD.**
**11-1, Ichinohashinomoto-cho,**
**Higashiyama-ku**
**Kyoto-shi 605(JP)**

(72) Inventor: **Tanaka, Keiji,**
**Sanyokasei-Kogyo-Kabushikikaisha**
**11-1, Ichinohashinomoto-cho,**
**Higashiyama-ku**
**Kyoto-shi 605(JP)**

(74) Representative: **Kador & Partner**
**Corneliusstrasse 15**
**W-8000 München 5(DE)**

(54) **Water absorbent mouldable resin composition.**

(57) A water absorbent mouldable resin composition comprising thermoplastic resin or solvent soluble resin and water absorbent copolymer resin dispersed therein. The water absorbent copolymer comprising a copolymer of a monomer represented by the formula [1], hydrophilic monomer and crosslinking agent if necessary, and have a water absorbency of 10 to 1000 g/g.

The resin composition may be mouldable into any shape, its absorbable water content is equal to 10 to 20000 wt% based on its own weight.

As the water absorbent copolymer resin have an excellent compatibility with the thermoplastic resin or solvent soluble resin, loss of hydrogel of the resin composition is maintained in a lower level.

[1]

$$CH_2=C(R_1)CO(OC_2H_4)_n(OC_3H_6)_mOR_2$$

($R_1$ :H or $CH_3$ , $R_2$ :long chain alkyl group, $n \geq 2$, $m \geq 0$)

EP 0 499 672 A1

Background of the invention

[Field of the invention]

The invention relates to a water absorbent mouldable resin composition.

[Description of prior art]

A conventional water absorbent resin is usually nonmouldable resin because of three dimensionally crosslinked high molecular type. For the purpose of moulding into any shape using such kind of resin, said water absorbent resin being prepared into smaller sized grain has to be made dispersed within a mouldable resin of any kind under the existance of compatibilizer such as surfactants.

Where to be mixed together through conventional procedure, the water absorbent resin was found less compatible with mouldable resin. In addition moulded procduts readily lose hydrogel, resulting in a fluctuation in water absorbability and a degradation of surface quality.

Summary of the invention

As a solution for the above problems, the invention is thus to be seen in the following description:

Namely, this invention relates to a water absorbent mouldable resin composition comprising thermoplastic resin or solvent soluble resin and water absorbent copolymer resin dispersed therein, wherein said water absorbent copolymer resin comprising a copolymer of a monomer represented by the following structural formula [1], hydrophilic monomer and crosslinking agent to be added therto when required, and have a water absorbency of from 10 to 1000g/g, wherein said resin composition may be mouldable into any shape, and have absorbable water content of to 10 to 20000% based on its own weight at the moulded state and possible loss of hydrogel of said water absorbent copolymer from moulded said water absorbent mouldable resin composition may be maintained to a level of less than 10% by weight at the equilibrated state in water content.

[1]

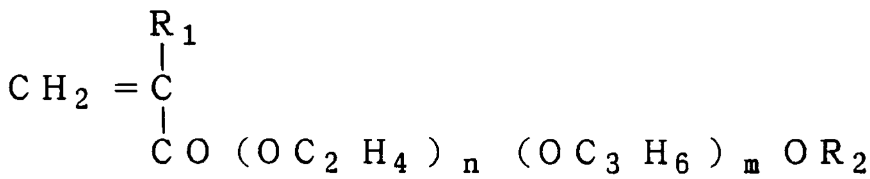

wherein:

- $R_1$ denotes H or $CH_3$ ;
- $R_2$ denotes long chain alkyl group having more than 5 carbon atoms;
- n denotes a positive number of 2 or more;
- m denotes 0 or a positive number.

Detailed description of the Invention

In the structural formula [1], n is preferably 2 to 50.

For the purpose of dispersion, n of less than 2 would give an adverse effect on the stability of reaction, so that the water absorbent copolymer resin could hardly be obtained. It is further noted that m should preferably be 0 to 20.

In addition, when $n \times OC_2H_4$ and $m \times OC_3H_6$ are to be used, they may be linked in any order in the structural formula [1]. Also either random- or block linkage may be employed.

The long chain alkyl group of $R_2$ may be either of linear-, branched- or cyclic type, provided that number of carbons should be 20 or be exceed 20 preferably.

For the purpose of dispersion operation, number of carbon atoms less than 5 would be given an adverse effect on the stability of reaction, so that the objective of the present invention could not be achieved.

While synthetic method to prepare monomer of formula [1] is not specifically defined here, it may be typically obtained through an esterification reaction of adduct of ethyleneoxide of higher alcohol and/or adduct of propyleneoxide of higher alcohal with (meth) acrylic acid and/or (meth)acrylic acid chloride. [hereinafter "(meth)acrylic" referred to as "acrylic" or "methacrylic"].

$$CH_2=\overset{\displaystyle R_1}{\overset{|}{C}}COOH \quad + \quad H(OC_2H_4)_nOR_2$$

$$CH_2=\overset{\displaystyle R_1}{\overset{|}{C}}CO(OC_2H_4)_nOR_2+H_2O$$

The hydrophilic monomer to be used under the present invention may be either of anionic vinyl-monomer, cationic vinyl-monomer, nonionic water soluble vinyl-monomer and mixtures thereof.

Anionic vinyl monomer may be of any type, but those which have carboxyl group and/or sulfon group are preferred.

Anionic vinyl-monomer having carboxyl group is typically an unsaturated mono- or polycarboxylic acid [for example, (meth) acrylic acid, ethacrylic acid, crotonic acid, sorbinic acid, maleic acid, itaconic acid, cinnamic acid, etc.], anhydride thereof [for example, maleic acid anhydride, etc.] and salt thereof [alkaline metal salt (salt of sodium, potassium, lithium, etc.), alkaline earth metal salt (salt of calcium, magnesium, etc.), ammonium salt and amine salt (salt of alkyl amine such as methyl amine, triethanol amine, diethanol amine, etc.; salt of alkanol amine such as triethanol amine, diethanol amine, etc.)] and mixtures from more than 2 of above described monomers.

Alkaline metal salts of (meth)acrylic acid are preferable.

Anionic vinyl monomer having sulfon group may be either of aliphatic- or aromatic vinyl sulfonic acids [vinyl sulfonic acid, allyl sulfonic acid, vinyl toluene sulfonic acid, styrene sulfonic acid, etc.], (meth)-acrylamide sulfonic acid [2-acrylamide-2-methylpropane sulfonic acid, etc.], or salts thereof [alkaline metal salt (salt of sodium, potassium, lithium, etc.), alkaline earth metal salt (salts of calcium, magnesium, etc.), ammonium- and amine salts (salt of alkyl amine such as methylamine, trimethylamine, etc.; salt of alkanol amine such as triethanolamine, diethanolamine)] and mixtures from more than 2 of above descrived monomers.

Alkaline metal salts of (meth)acrylamide sulfonic acid are preferable.

As cationic vinyl monomer, any type of cationic monomers may be used, but those having group of quaternary ammonium salt are preferred. Cationic vinyl monomer having group of quaternary ammonium salt may be either of reactant from dialkylaminoalkyl-(meth)acrylate and alkyl halide or dialakyl sulfonic acid [e.g. (meth)acryloiloxyethyltrimethylammonium chloride or bromide, (meth) acryloiloxyethyltrimethylammoniummetho-sulfate, (meth)acryloiloxyethyldimethylethylammonium chloride, (meth)acryloiloxyethyldiethylmethylammonium chloride, (meth)acryloiloxyethyldimethylbenzilammonium chloride, (meth)acryloiloxypropyltrimethylammonium chloride, (meth)-acryloiloxypropyltrimethylammoniumetho-sulfate, etc.]; reactant from dialkylaminohydroxyalkyl (meth)-acrylate and alkyl halide or dialkyl sulfuric acid [e.g. (meth)acryloiloxyhydroxyethyltrimethylammonium chloride or -bromide, (meth)acryloiloxyhydroxyethyltrimethylammoniummethosulfate, (meth)-aryloiloxyhydroxypropyltrimethylammonium chloride, etc.]; reactant from dialkylaminoalkyl-(meth)acrylamide and alkyl halide or dialkyl sulfuric acid [e.g. chloride or bromide of trimethylaminoethyl-(meth)acrylamide, chloride of trimethylaminopropyl-(meth)acrylamide, chloride of diethylmethylaminopropyl-(meth)acrylamide, etc.]; reactant from dialkylaminohydroxyalkyl-(meth)acrylamide and alkyl halide or dialkyl sulfuric acid [e.g. chloride of trimethylaminohydroxyethyl-(meth)acrylamide, chloride of trimethylaminohydroxypropyl-(meth)-acrylamide, chloride of diethylmethylaminohydroxypropyl-(meth)acrylamide, etc.]; N-alkylvinylpyridinium halide [e.g. N-methyl-2-vinylpyridinium chloride or -bromide, N-methyl-4-vinyl-pyridinium chloride, etc.], trialkylallylammonium halide [e.g. trimethyl-allylammonium chloride or bromide, triethylammonium chloride, etc.] or mixtures of more than 2 of the above described monomers. Reactants from dialkylamino-(meth)-acrylate and alkyl halide are preferable.

Example of nonionic water soluble vinyl monomer are hydroxyethyl-(meth)acrylate, hydroxypropyl-(meth)acrylate, (meth)acrylamide, vinylpyrolidone, etc.

For the purpose of obtaining a crosslinked polymer, crosslinking agent may be used. As the crosslinking agent, the compound (1) having at least 2 polymerizable double bonds or the compound (2) having one polymerizable double bond and at least one group which is reactive with the functional group of cationic-and/or anionic vinyl monomer may be preferable.

Examples of the crosslinking agent of the compound (1) are:

(1) bis-(meth)acrylamide: e.g. N,N-alkylene (having 1-6 carbon atoms)-bis-(meth)acrylamide such as N,N-methylen-bis-acrylamide etc.

(2) di- or polyester from polyols and unsaturated mono- or polycarboxylic acid: di- or tri-(meth)acrylic-acid ester of polyols [such as ethylene glycol, trimethylol-propane, glycerin, polyoxyethylene glycol, polyoxypropylene glycol, etc.]; unsaturated polyester [obtainable through the reaction of polyols such as polyols mentioned above with unsaturated acid such as maleic acid, etc.], di- or tri-(meth)acrylic acid ester [obtainable through the reaction of polyepoxide with (meth)acrylic acid], etc.

(3) Carbamyl ester: those which are obtainable through the reaction of polyisocyanate [such as tolylene diisocyanate, hexamethylenediisocyanate, 4,4'-diphenylmethanediisocyanate, prepolymer with NCO-group (which is obtainable through reaction of polyisocyanate with a compound having active hydrogen atoms), etc.] with hydroxyethyl (meth)acrylate.

(4) Di- or polyvinyl compound: divinylbenzene, divinyltoluene, divinylxylene, divinylether, divinylketone, trivinylbenzene, etc.

(5) Di- or poly-(meth)allylether of polyols:
for instance, polyethylenglycol-diallylether and allylated starch, allylated cellulose. [wherein illustrative polyol is, for instance, alkyleneglycol, glycerin, polyalkylene glycol, polyalkylene-polyol, carbohydrate, etc.]

(6) Di- or polyallylester of polycarboxylic acid:
diallylphthalate, diallyladipate, etc.

(7) Ester from unsaturated mono- or polycarboxylic acid and mono-(meth)allylether of polyol: (meth)-acrylic acid ester of polyethyleneglycolmonoallylether, etc.

(8) Polyallyloxylalkanes: tetraallyloxyethane, etc.

Examples of the compound (2) are: ethylenically unsaturated compound having reactive groups with carboxyl-, sulfon- and/or quaternary ammonium salt group. Example of such reactive groups are, for instance, hydroxyl-, epoxy- or tertiary amino-group, etc.

Specific examples of the compound (2) are unsaturated compound with hydroxyl-group [such as N-methylol (meth)acrylamide, etc.], unsaturated compound with epoxy-group [glycidyl (meth)acrylate, etc.], unsaturated compound with tertiary amino-group [such as dimethylaminoethyl (meth)acrylate, diethylaminoethyl (meth)acrylate, etc.], etc.

As these crosslinking agents, the compound (1) is preferred, particularly N,N'-methylen-bis-acrylamide, ethyleneglycoldiacrylate, trimethylolpropanetriacrylate and tetraallyloxyethane are more preferred.

Weight ratio of the monomer under [1] to the hydrophilic monomer is usually 1:99 to 80:20. A proportion of less than 1 will cause an instability of polymerization.

Conversely where 80 were exceeded, the product would become less hydrophilic, causing its usage to be inevitably limited.

Using amount of the crosslinking agent is normally 0.01 to 10 wt%, preferably 0.1 to 5 wt% to the total weight of the monomer represented by the formula [1] and the hydrophilic monomer.

As polymers from these types of monomer,crosslinked polymer from the monomer represented by the formula [1] and sodium acrylate, or crosslinked polymer from the monomer represented by the formula [1] and poly-2-acrylamide-2-methylpropane sulfonic acid may be examplified.

The composition of water absorbent copolymer resin can vary depending on its intended application - for a product with higher absorbency, anionic crosslinked polymer is suitable, and for a product with salt resistance and water absorbency, amphoteric crosslinked polymer is preferred.

For the purpose of polymerization of hydrophilic monomer, reverse phased emulsion polymerization or reverse phased suspension polymerization is more preferable. Generally, in the reverse phased emulsion polymerization or the reverse phased suspention polymerization of hydrophilic monomers, a hydrophilic monomer solution by water is emulsified or suspended in a hydrophobic medium. While solution polymerization is made available, through which the monomer represented by the formula [1] is made soluble into hydrophilic monomer solution in water, however, it is difficult to grain the obtained polymer and to distribute long chain alkyl groups effectively over the grain surface of the products obtained thereby.

In carrying out reverse phased emulsion polymerization or reverse phased suspension polymerization, the monomer represented by the formula [1] may be made in use through making it soluble within hydrophobic medium or hydrophilic monomer solution in water.

Generally those polymerisation catalysts available for reverse phased emulsion polymerization are oil-soluble, whereas those for reverse phased suspension polymerization are water soluble.

Oil-soluble polymerization catalysts may be either of azo-type catalysts such as azobisisobutyronitrile, etc., or organic peroxide type catalysts such as isobutylperoxide, etc.

Water soluble polymerisation catalysts may be either of azo-type catalysts such as azobisamidinopropanedihydrochloride, etc., organic peroxide type catalysts such as t-butylpermaleic acid, etc., inorganic peroxide type catalysts such as potassium persulfate or other redox type catalyzer, etc.

Hydrophobic medium available for reverse phased emulsion polymerization or reverse phased suspension polymerization may be either of aliphatic-, aromatic-, alicyclic-, halogenated hydrocarbon, mineral oil or any type of hydrophobic medium. Aliphatic- or alicyclic hydrocarbons are preferable.

Usually emulsifier or dispersant is not required for the reverse phased emulsion polymerization or the reverse phased suspension polymerization, but usual type of such emulsifier or dispersant may be used where appropriate.

For this purpose, sorbitanmonostearate, hydroxyethylcellulose, etc., are considered useful.

Dehydration of water containing water absorbent copolymer resin or separation of hydrophobic medium from the copolymer resin may be made utilizing conventional method, either of azeotropic dehydration under the state of emulsion or suspension within hydrophobic medium followed by centrifugation or filtration, or after separation of the obtained polymer particle from hydrophobic medium followed by drying using hot air or vacuum, spray drying or refrigeration drying. Averaged particle size of dried water absorbent copolymer resin should be as small as possible, preferably less than 10μm, more preferably less than 1μm.

To reduce particle size dwon to less than 1 μm, the reverse phased emulsion polymerization should preferably be adopted, wherein single run of process operation is considered enough to obtain particle size as such.

For particle size reduction in case of polymer which has been obtained through other type of procedure, mechanical means such as jet mill, ultrasonic homogenizer, etc., are useful.

The water absorbent copolymer resin to be made dispersed should not necessarily be maintained under dry state, but is allowed to be in a moistened state, as long as no adverse effect will result in moulding.

Water absorbency of these copolymer resins are ranged from 10 to 1000 g/g and perferably from 100 to 1000 g/g.

Referring to the thermoplastic resins or solvent soluble resins, they are not specifically defined and any type resins available in the market will serve well for the purpose intended, so long as they are adequately mouldable for moulding process. Polyolefin resins [e.g. polyethylene, polypropylene, polyvinyl chloride, chlorinated polyethylen, ethylene-vinyl acetate copolymer (EVA), maleicated EVA, ethylene-(meth)acrylic acid copolymer, ethylene-(meth)acrylic acid ester copolymer, polystylene, polyvinylalcohol (PVA), etc.], polyester resins [e.g. polyethyleneterephthalate, etc.], polyacrylonitrile resins, polyurethane resins, polyamide resins, rubbers [e.g. synthetic rubbers such as butyl rubber, isoprene rubber, butadiene rubber, chloroprene rubber, stylene-butadiene copolymeric rubber and natural rubber], natural resin [e.g. beeswax, tallow etc.], fatty acid ester with long alkyl chain, adduct of polyalkylene oxide [e.g. polyethyleneglycol, ethylene oxide adduct of higher alkyl alcohol etc.] are illustrated as typical examples.

These resins may be made available in single or mixed together depending on the purpose intended.

Word "solvent" which is mentioned above, means any kind of solvent which is capable to make resin dissolved. Aromatic solvent [benzene, toluene, xylene, etc.], hydrocarbon type solvent [hexane, cyclohexane, etc.]. polar solvent [water, acetone, methanol, N,N-dimethylformamide (DMF), dimethylsulfoxide (DMSO), etc.] are considered typical.

Weight ratio of water absorbent copolymer resin to thermoplastic or solvent soluble resin may be fixed depending on the absorbency and mouldability and usually ranged from 3:97 to 80:20.

If the ratio of the water absorbent copolymer resin were less than 3, the absorbency would become remarkably reduced, in where 80 were exceeded, it is inferior in the mouldability.

As a typical example of the mouldable water absorbent resin composition of this invention, EVA in which crosslinked copolymer of sodium acrylate and monomer represented by the formula [1] is made dispersed or maleicated EVA in which crosslinked copolymer of poly-2-acrylamide-2-methylpropane sulfonic acid and monomer represented by the formula [1] is made dispersed may be raised.

Referring now to the dispersing method of water absorbent copolymer resin within thermoplastic resins or solvent soluble resins, following procedures are found typical;

(1) Water absorbent copolymer resin emulsion upon completion of reverse phase emulsifying polymerization is made dispersed within thermoplastic resins or solvent soluble resins which is heat melted

with the aid of solvent when required. And then solvent is removed.

(2) Water absorbent copolymer resin obtained through reverse phase emulsifying polymerization or other polymerization and disintegration into a fine particles is made dispersed within thermoplastic resins or solvent soluble resins which are heat melted with the aid of solvent when required. And then solvent is removed if necessary.

(3) Water absorbent copolymer resin particles or powder with an averaged particle size less than 1 $\mu$m and thermoplastic resins pellet are intermixed, so as to be blended thereupon.

The above dispersion process may be taken place before the moulding process into any shape [e.g. pellet, bar, sheet, etc.] or both processes may be taken place concurrently.

Composition disclosed under the present invention may be made moulded into any of powder, pellet, bar, film, sheet, fiber, etc.

No specific methods are defined under the present invention for the moulding process, but ordinary processes [e.g. granulator, pelletizer, melting extruder, calender roll, casting machine, etc.] may be made available for (1) moulding thermoplastic resin or solvent soluble resin containing water absorbent copolymer resin particles being dispersed therein, or (2) causing water absorbent copolymer resin to disperse in thermoplastic resin or solvent soluble resin and moulding thereof concurrently.

Water absorbent copolymer resin and thermoplastic resin or solvent soluble resin disclosed under the present invention may be added with surfactants, orgnic powder materials (e.g. pulp-powder, starch, cellulose derivative, natural polysaccharide, etc.) and inorganic powder materials (e.g. permilite, zeolite, silica, permiculite, bentonite, talc, activated carbon, etc.) as fillers or additives in a suitable amount if necessary.

In addition anti-oxydation agent, plasticizer, mold preventive agent, dyestuff, pharmaceutical component, perfume, etc. may be added thereto.

Amount of these agent is normally less than 10 weight % of the total weight of mouldable water absorbent composition.

The moulded articles of the mouldable water absorbent resin composition of this invention should have a water absorbency of 10 to 20000% based on the own weight at the moulded state.

Such absorbency less than 10% would cause the use of products to be inevitably limited. Conversely where 20000% were exceeded, the strength of the products is too low to use in the state of water-absorption. The possible loss of hydrogel of water absorbent copolymer resin from the moulded products under equilibrated state of water shall be less than 10% by weight.

Exceeding 10% of the possible loss of the hydrogel would adversely affect about surface feeling such as slippery feeling. The monomers represented by the formula [1] form a monomer micelle phase which is stable in polymerization in progress since the monomer represented by the formula [1] has both segments, one having an affinity for water and the other having an affinity for oil. In light of this, it may be surmised that the obtained copolymer particles would have long chain alkyl group uniformly distributed over their entire surface. This could reasonably explain why the water absorbent resin may be effectively fixed within the mouldable resin in such a manner that the long chain alkyl group and hydrophobic segment of the mouldable thermoplastic- or solvent soluble resin would become soluble among each other while in moulding operation.

[Examples]

The present invention is described hereunder with reference to some examples of manufacturing procedures and embodiments but with the understanding that the present invention is not specifically limited thereto. The word "part" means here a part in weight.

[Example of manufacturing procedure]

The particle size of the water absorbent copolymer resin was measured through the wet-type super centrifugal precipitation method (using super centrifugal precipitator "CAPA-7000" manufactured by Horiba Seisakusho). The water absorbency of the water absorbent copolymer resin was determined by the following procedure:

0.1g of the copolymer resin were put into 100 ml beaker and were allowed to absorb ion-exchanged water for 1 hr. Fine particle gels containing water as water dispersion was subjected to centrifugation so as to separate it into lower gel layer and upper surplus water layer.

The water absorbency in equation is then equal to 10 times of the quantity of gel layer yielded.

Preperation Example 1

3 parts of monomer (A) under formula [1] wherein $R_1$ is H, n is 20, m is 0 and $R_2$ is linear alkyl group having 20 carbon atoms respectively, which were prepared using adduct of ethyleneoxide with higher alcohol and acrylic acid through esterificaiton reaction, and 40 parts of cyclohexane were taken into an agitatable reactor for nitrogen replacement under 50 °C for 1 hr. with the monomer (A) being made soluble.

4 parts out of 16 parts of 45 wt% solution of sodium acrylate (nuetralized at 74 mol%) in water containing methylenebisacrylamide (0.2 wt% to acrylic acid) were put into the reactor under agitation. 0.001 parts of azobisisobutylonitrile were then added thereto. 30 minutes later, the remnant of the said sodium acrylate solution in water was allowed to drop thereon for 1.5 hrs. and then it was aged under 60°C for 1 hr.

Through subsequent azeotropic dehydration under 70 to 80°C, the cyclohexane solution of the emulsion (B) with crosslinked copolymer of sodium acrylate being dispersed therein was thus yielded.

Solid component of the water absorbent copolymer resin in the emulsion (B) is 25.5 wt%; averaged particle size of the water absorbent resin is 0.2µm.

The emulsion (B) was then subjected to ultra-centrifugation to separate it into solvent and resin solid. The water absorbency of said resin solid measured to 400 g/g.

Preparation Example 2

Same polymerization as in the preparation example 1 took place except that instead of 16 parts of 45% solution of sodium acrylate (neutralized at 74 mol%) in water, 10 parts of 60 wt% solution in water of mixture of acryloiloxyethyltrimethylammonium chloride containing methylenebisacrylamide (0.2 wt% to monomer) and acrylic acid (molar ratio is 1:1) was used here.

The cyclohexane solution of the emulsion (C) with amphoteric crosslinked polymer being dispersed therein was thus yielded.

Solid component of the water absorbent copolymer resin in emulsion (C) is 20 wt%, averaged particle size of the water absorbent copolymer resin is 0.5µm.

The emulsion (C) was then subjected to ultra centrifugation to separate it into solvent and resin solid.

The water absorbency of the resin solid measured to 250 g/g.

Preparation Example 3

A monomer mixture was prepared in such a manner that 3 parts of monomer (D) under formula [1] wherein $R_1$ is H, n is 30, m is 1 and $R_2$ is linear alkyl group having 16 carbon atoms respectively, which were synthesized by esterification from an adduct of ethyleneoxide of higher alkyl alcohol, an adduct of propylene oxide of higher alkyl alcohol and an acrylic acid, were admixed into 16 parts of 45% solution of sodium acrylate (neutralized at 74 mol%) containing methylene-bis-acrylamide (0.2wt% to the acrylic acid) in water through solubilation.

30 parts of cyclohexane were transfused into an agitatable reactor for nitrogen replacement under 50 °C for 1 hr. 5 parts out of 19 parts of said monomer mixture were added thereto under agitation. 0.001 parts of azobisisobutylonitrile were further added. 30 minutes later, the remnant of said mixture was allowed to drop thereon for 1.5 hrs for subsequent aging under 60°C for 1 hr.

Through azeotropic dehydration under 70 to 80 °C, cyclohexane solution of the emulsion (E) with cross-linked polymer of sodium acrylate being dispersed therein was thus yielded.

Solid component of the water absorbent copolymer resin in the emulsion (E) is 25.5 wt%; averaged particle size of the water absorbent copolymer resin is 0.2µm.

The emulsion (E) was then subjected to ultra-centrifugaion to separate it into solvent and resin solid.

The water absorbency of said resin solid measured to 380 g/g.

[Example]

The water absorbency of water absorbent mouldable resin composition was measured using weight variation method. For this purpose 5g of specimen in a Nylon tea-bag of 250 mesh were kept immersed within ion-exchanged water for 4 hrs. The Nylon tea-bag taken out of water, upon removal of excess water for 15 minutes, was weighed. 1/5 × 100 of the weight increment is referred to as water absorbency (in % with reference to own weight).

Possible loss of hydrogel of the water absorbent copolymer resin occurring while in water absorption with the water absorbent resin composition under the present invention was evaluated through evaporation

residue method. For this purpose, the specimen, upon determination of water absorbency through weight variation method, was filtrated using a stainless steel wire net of 80 mesh. The resulted filtrate was made into dry substance to determine residual weight. The loss of hydrogel resulted was evaluated using the following equation:

Loss of hydrogel (%) = weight of residual solid × (100/5) × weight of solid content of water absorbent copolymer resin in the water absorbent moulding resin composition

Example 1

300 parts of toluene and 80 parts of low density polyethylene (product by Mitsubishi Petrochemical Co., Ltd. "LED polyethy") were dissolved in a vessel under 80 ° C.

130 parts of emulsion (B) were allowed to drop thereon so as to become mixed and dispersed therein.

The resulted suspension was allowed to be spread over a separation paper. Drying and subsequent disintegration then followed.

Compound (F) with the water absorbent copolymer resin being dispersed in polyethylene under the present invention was thus yielded. A film obtained through inflation moulding by brabender moulder of compound (F) is shown in table 1 in term of water absorbency and loss of hydrogel.

Example 2

Through the same procedure as in the Example 1 except that 260 parts of emulsion (B) were used instead of 130 parts of emulsion (B), water absorbent mouldable resin composition (G) under the present invention was yielded. The water absorbency and loss of hydrogel of a film obtained through inflation moulding by brabender moulder of the compound (G) is shown in table 1.

Example 3

300 parts of toluene and 80 parts of ethylene-vinyl acetate copolymer (EVA) (manufactured by Mitsubishi Petrochemical Co., Ltd. "Polyethy EVA 25K") were dissolved completely in a vessel under 80 ° C. 200 parts of emulsion (C) were allowed to drop thereon so as to become mixed and dispersed therein.

The resulted suspension was allowed to be spread over a separation paper. Drying and freeze-granulating then followed. Compound (H) with the water absorbent copolymer resin being dispersed in EVA under the present invention was thus yielded. The water absorbency and loss of hydrogel of a film obtained through inflation moulding by brabender moulder of the compound (H) is shown in table 1.

Example 4

300 parts of toluene and 80 parts of ethylene-vinyl acetate (EVA) (manufactured by Mitsubishi Petrochemical Co., Ltd. "Polyethy X700") were dissolved in a vessel under 80 ° C . 200 parts of emulsion (C) were allowed to drop thereon so as to become mixed and dispersed therein.

The resulted suspension was allowd to be casted on a separation paper and then dried. Composition (I) with the water absorbent copolymer resin being dispersed in EVA film under the present invention was thus yielded.

The water absorbency and loss of hydrogel of the resulted film is shown in Table 1.

Example 5

Through the same procedure as in the example 4 except that 120 parts of emulsion (E) were used instead of 200 parts of emulsion (C), water absorbent resin composition (I) was yielded.

The resulted film is shown in table 1 in term of water absorbency and loss of hydrogel.

Example 6

400 parts of dimethylsulfoxide and 80 parts of polyvinylalcohol (PVA) (manufactured by Kuraray Co., Ltd. "Poval 217") were completely dissolved in a vessel and 200 parts of emulsion (E) were allowed to drop

thereon so as to be mixed and dispersed therein.

The resulted suspension was allowed to be casted on a separation paper and then dried. Composition (J) with the water absorbent copolymer resin being dispersed in PVA sheet under the present invention was thus yielded.

The water absorbency and loss of hydrogel of the PVA sheet resulted through heat treatment and crosslinking of the composition (J) is shown in table 1.

Comparative example 1

Through the same procedure as in the Example 3 except that 38 parts of "SANWET IM-300SP" (water absorbent copolymer resin, manufactured by Sanyo Chemical Industries, Ltd.) were used instead of 200 parts of emulsion (C) the product (K) with the water absorbent copolymer resin being dipersed in EVA film was yielded.

The water absorbency and loss of hydrogel of the resulted film is shown in Table 1.

Table 1

| Specimen | Water absorbency(%) | Loss of hydrogel(%) |
|---|---|---|
| Example 1 | 19 | 0.08 |
| Example 2 | 47 | 0.15 |
| Example 3 | 35 | 0.10 |
| Example 4 | 520 | 0.40 |
| Example 5 | 490 | 0.90 |
| Example 6 | 1250 | 1.30 |
| Comparative example 1 | 30 | 15.50 |

[Effect of the invention]

The mouldable water absorbent resin composition under the present invention provides following advantages;

(1) It is mouldable in any type of shape.

(2) Under the state of the moulded shape, it shows an excellent water absorbency, because the water absorbent copolymer resin have higher potency to be compounded within moulding resin.

(3) Due to a high potency to be compounded within moulding resin, loss of hydrogel upon absorption of water is maintained in a lower level, so that stable water absorbency is kept and feeling of surface or safety to human skin is improved.

(4) Gel-strength upon absorption of water is higher than that of pure water absorbent copolymer resin itself.

Based on the aforementioned advanteges, it may be made available in various applications, e.g. film used in agriculture or foodstuff, water barrier for civil engineering field, water absorbent materials such as water absorbent fiber, toy, sanitary napkin, concrete curing sheet.

Cross-linked high molecular compound with anionic, cationic group or amphoteric compound contains in itself ion group. Accordingly hydrogel thereof releases the absorbed water when electric potential is applied and becomes contracted. In contrast, it will absorb water and become swelled when electric potential removed. The above response can occur repeatedly (Y. OSADA. Adv. Polym. Sci,. 82, 1 (1987)).

The mouldable water absorbent resin composition will function as aforesaid, when it contains ion-group and hence be applicable to those applications which rely on the aforementioned function (e.g. dehydration sheet for sludge processing, artificial muscle, actuator, solar pond, etc.)

## Claims

**1.** A water absorbent mouldable resin composition comprising thermoplastic resin or solvent soluble resin and water absorbent copolymer resin dispersed therein, wherein said water absorbent copolymer resin

comprising a copolymer of a monomer represented by the following structural formula [1], hydrophilic monomer and crosslinking agent to be added therto when required, and have a water absorbency of from 10 to 1000g/g, wherein said resin composition may be mouldable into any shape, and have absorbable water content of 10 to 20000% based on its own weight at the moulded state and possible loss of hydrogel of said water absorbent copolymer from moulded said water absorbent mouldable resin composition may be maintained to a level of less than 10% by weight at the equilibrated state in water content.

[1]

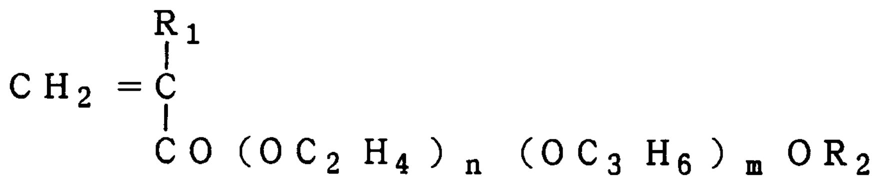

wherein:

- $R_1$ denotes H or $CH_3$ ;
- $R_2$ denotes long chain alkyl group having more than 5 carbon atoms;
- n denotes a positive number of 2 or more;
- m denotes 0 or a positive number.

2. A water absorbent mouldable resin composition set forth in Claim 1, wherein said water absorbent copolymer resin is obtainable through polymerization such as reverse phased emulsion polymerization or reverse phased suspension polymerization in such a manner that the monomer represented by the structural formula [1] is dissolved within a hydrophobic medium.

3. A water absorbent mouldable resin composition set forth in Claim 1, wherein said water absorbent copolymer resin is obtainable through polymerization such as reverse phased emulsion polymerization or reverse phased suspension polymerization in such a manner that the monomer represented by the structural formula [1] is dissolved within a hydrophilic medium.

4. A water absorbent mouldable resin composition set forth in any claim of 1 to 3, wherein said water absorbent copolymer resin comprises at least one crosslinked high molecular compound selected from the group consisting of a crosslinked high molecular compound having anionic group, a crosslinked high molecular compound having cationic group, a nonionic crosslinked high molecular compound and an amphoteric crosslinked high molecular compound having both of anionic group and cationic group.

5. A water absorbent mouldable resin composition set forth in any claim of 1 to 4, wherein said composition have moulded shape of any of powder, pellet, bar, film, sheet or fiber.

6. A water absorbent mouldable resin composition set forth in any claim of 1 to 5, wherein said water absorbent copolymer resin may be mixed with thermoplastic resin or solvent soluble resin in a ratio of 3:97 to 80:20 in weight.

European Patent Office

# EUROPEAN SEARCH REPORT

Application Number

EP 91 10 2534

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 157 928 (DAINIPPON INK & CHEMICALS, INC.) * Claims; page 7, line 35 - page 8, line 4 * | 1 | C 08 L 33/14<br>C 08 F 220/28 |
| X | EP-A-0 011 806 (THE DOW CHEMICAL CO.) * Claims; page 16, line 7 - page 18, line 18 * | 1-6 | |
| A | EP-A-0 003 235 (BASF AG) * Claims * | 1 | |

| TECHNICAL FIELDS SEARCHED (Int. Cl.5) |
|---|
| C 08 L<br>C 08 F |

The present search report has been drawn up for all claims

| Place of search | Date of completion of the search | Examiner |
|---|---|---|
| THE HAGUE | 29-10-1991 | DE LOS ARCOS Y VELAZQUEZ |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)